# EUROPEAN PATENT APPLICATION

(11) **EP 2 930 513 A1**
(43) Date of publication of application: **14.10.2015**
(21) Application number: 14164482.3
(22) Date of filing: 11.04.2014
(51) Int. Cl.: G01N 33/49

(54) **Removal of particulates from blood using an apparatus including a size-differentiating element**

(30) Priority: 09.04.2014 US 201414248416
(71) Applicant: Parsortix, Inc., Philadelphia, PA 19104-5504 (US); Hvichia, George, Philadelphia, PA 19116 (US)
(72) Inventor: Hvichia, George, Philadelphia, PA Pennsylvania 19116 (US)
(74) Representative: Bond, Christopher William

(57) **Abstract**

The invention relates to methods of using an apparatus to sequester thrombi (including emboli) from blood of an animal such as a human. The apparatus comprises a stepped or sloped separation element interposed between an inlet region and an outlet region of a void that can be filled with fluid. The void can be enclosed within a cover and fluid flow through the void engages cells and thrombi/emboli in a blood sample with the separation element. Only particles (e.g., blood cells) which have or can deform to have a characteristic dimension smaller than or equal to the distance between a step and the cover or body can pass onto or past a step. By selecting dimensions which permit blood cells, but not thrombi or emboli, to pass onto or past a step and passing fluid through the apparatus, thrombi/emboli can be sequested from the blood sample. The thrombi can be observed, removed, or treated to effect their degradation or lysis.

## Description

### BACKGROUND OF THE INVENTION

A property of the blood of most, if not all, animals is the ability to form clots or thrombi. A thrombus is a conglomeration of blood cells and extracellular materials that forms a solid or semi-solid mass. Generally formed through specific interactions among blood platelets and humoral clotting factors, thrombi can also entrap red and white blood cells that are present on or within the matrix of a thrombus during its formation. Thrombi can form relatively rapidly, owing to a well-known cascade of thromboregulatory factors and reactions which promote or inhibit platelet aggregation and cross-linking among clotting factors and platelets. The thrombotic cascade

Thrombus formation (thrombosis) is a normal and beneficial response to physical injury involving inappropriate flow of blood from a blood vessel or a tissue. For exterior injuries, such as cuts and scratches in the skin, the thrombus can be externally visible as a scab that forms to occlude breaches that extend through the skin from which systemic blood could flow in the absence of the thrombus. Thrombi can form within the body as well, such as within blood vessels or on or within surfaces of vascularized tissues.

Not all thrombosis is beneficial, and some forms of thrombosis can be detrimental to health or even fatal. Thrombi attached within the lumen of a blood vessel can narrow or even occlude the vessel, reducing blood flow therethrough. A thrombus attached within a blood vessel can also detach from its site of attachment or shed pieces of the thrombus into the bloodstream, and these detached thrombus pieces are generally referred to as emboli. An embolus can pass through and along a blood vessel so long as it has a size small enough to flow through the lumen of the vessel, and so long as it does not attach itself to a wall of the vessel or a material (e.g., an arterial plaque) within the vessel.

Mere passage of an embolus through a blood vessel tends to be non-pathogenic (unless the embolus attaches within its lumen). However, emboli which are unable to pass through a blood vessel at the same rate as blood will partially or completely occlude blood flow through that vessel. Tissues which draw their nutrition, oxygen supply, or both from a thrombus- or embolus-occluded blood vessel can become malnourished or ischemic if the occlusion persists. Although drugs exist which are known to induce or accelerate thrombolysis, the relatively short time span (tens of minutes to hours or tens of hours) during which even limited ischemia or malnutrition can endure prior to administration of these drugs can result in serious pathological damage to sensitive tissues such as brain or cardiac muscle, resulting in serious injury or death. No clear boundary exists between thrombi and emboli, in that thrombi can sometimes 'slip' or move slowly along a blood vessel without necessarily becoming a freely-circulating embolism and an embolism that is slowed within or transiently attaches to a blood vessel can grow in place, gradually or rapidly losing its ability to move with blood flow.

Several serious pathologies result from thrombosis and/or subsequent embolization.

Stroke is a term generally applied to formation of a thrombus within an blood vessel which supplies cerebral tissue or occlusion of such a vessel by an embolism that becomes trapped within such a vessel. Cerebral blood vessel occlusion can arise from thrombi which arise in situ, from vessel-occluding emboli formed from non-cerebral thrombi and carried by the blood to the vessel, and from non-cerebrally-generated emboli which are initially too small to occlude vessels, but which grow and/or attach within cerebral blood vessels. A variety of factors (e.g., hypertension, occurrence of atherosclerosis or inflammatory disease within a patient, and habitual smoking) are known to increase the likelihood of stroke. Significantly, the period between the occurrence of cerebral blood vessel occlusion and the onset of stroke symptoms (which can sometimes be difficult to detect) can be as short as seconds, minutes, or tens of minutes, and the delay between onset and irreversible cerebral ischemic injury can be as short as hours or tens of hours. Owing to the brevity of these time periods, it is often difficult to administer thrombolytic drugs to patients experiencing scope soon enough to prevent permanent or serious injury.

Transient ischemic attacks (TIAs) are similar to stroke in that they involve detectable decrease in cognitive and/or motor functions arising from ischemia that results from cerebral blood vessel occlusion. TIAs are generally distinguished from strokes in that TIAs result in little or no permanent ischemic damage to cerebral tissue. Nonetheless, the boundary between a TIA and a stroke is not a clear one. At least some TIAs are believed to result from occlusion induced by thrombi or emboli.

Pulmonary embolism (PE) is a condition in which a thrombus or embolus occludes blood circulation through a major blood vessel in the lung. PE can result in deficient blood oxygenation (with myriad body-wide) consequences, including circulatory instability. PE is also a leading cause of sudden death. Like stroke, PE can arise from a thrombus that grows in situ, but (also like stroke) it more frequently arises from embolization in a non-lung tissue (e.g., within a femoral vein) and lodging of the resulting embolus in a lung artery. Also like stroke, the period of time that elapses between occurrence of a PE and serious tissue damage or death can be very short - on the order of minutes or hours - potentially precluding its effective treatment. Thus, prevention of PE can be of far greater health significance than treatment of existing PE.

Cardiac embolism (CE) involves occlusion of a coronary artery by a thrombus or (more frequently) an embolism, such as an embolus detached from a left ventricular thrombus. Symptoms of cardiac embolism resemble those of cardiac infarction (some infarctions arise directly from lodging of an embolus within a coronary artery) and include weakness and chest pain. Like PE and stroke, the lag time between onset of CE and infliction of serious or irreversible injury can be very short - generally on the scale of minutes to hours.

The factors which affect thrombosis, embolization, and thrombolysis are not fully understood. Nonetheless, certain patients are known to be at enhanced risk of developing thrombosis and emboli. For example, human patients whom have previously been afflicted with a thrombotic or embolic disorder (e.g., stroke, TIA, CE, or PE) are known to be at risk of future episodes. Similarly, certain behaviors (e.g., smoking, prolonged inactivity such as excessive bed rest or remaining seated during long-duration airline flights) and characteristics (e.g., hypertension, affliction with acute or chronic inflammatory disorders or vascular endothelial disorders) are known to increase the likelihood of thrombus and embolus occurrence in individuals. For such patients, the devices and their uses described herein may be particularly beneficial.

Bodily sites of thrombus formation and embolization are generally known. For example, thrombosis and embolization are known to occur in large arteries (e.g., the aorta) and the left cardiac atrium of patients afflicted with atrial fibrillation and in femoral (and other) veins of patients afflicted with deep vein thrombosis. Thus, although the physiological site(s) of thrombosis and embolization often cannot be predicted with certainty for any given patient, sites of common thrombus- and embolus-formation are known.

Thrombotic disorders can be difficult to diagnose prior to infliction of significant ischemic damage upon a patient. In part, this difficulty results from the elusive nature of thrombi and emboli. Thrombi and emboli can form, move, and dissolve within a patient's bloodstream without detection by the patient, by individuals observing a patient, or even by investigators who seek to detect them (unless they fortuitously observe a sample including a thrombus or embolus). It would be beneficial if thrombi and/or emboli could be detected within the blood circulation of an individual at a time sufficiently prior to corresponding physiological damage that the disorder or condition that contributes to thrombus/embolus formation can be diagnosed or treated.

Once formed within the body, a thrombus or embolism does not necessarily endure forever. Instead, natural thrombolytic factors present or synthesized within the body (especially humoral thrombolytic factors such as plasminogen and various proteases) induce degradation of thrombi and emboli, reducing their size and rendering them incapable of occluding blood vessels.

It would be desirable if thrombotic clumps or emboli could be sequestered from circulating blood at a position upstream from a sensitive tissue, rather than (or in addition to) relying upon prompt administration of thrombolytic drugs to a patient after ischemic injury to the sensitive tissue has begun. Even if not physically removed from the blood stream, emboli or thrombi that are sequestered upstream from a sensitive tissue will not occlude blood vessels within the tissue and can be acted upon by naturally-occurring (or administered) thrombolytic factors to degrade them sufficiently that their degradation products will not occlude vessels in the tissue.

The subject matter disclosed herein addresses the issues referred to above.

### BRIEF SUMMARY OF THE INVENTION

The invention relates to a method of sequestering a thrombus in a blood sample. The method includes providing the blood sample to the inlet region of an apparatus and passing a fluid from an inlet region to an outlet region of the apparatus, with the thrombus being sequestered between the inlet and outlet regions. The apparatus includes a body, a cover, and a separation element. The body and cover define a void having an inlet region, an outlet region, and a surface. The body and cover can be separate entities or a single unitary entity bent or folded back upon itself. The separation element i) is disposed in the void, ii) has at least one step including a first step, and iii) defines a narrow passageway that fluidly connects the inlet and outlet regions of the device. The narrow passageway includes at least a first passageway that is bounded by the first step and the surface of the void.

The height of the first passageway i) is defined by the distance between the first step and the surface of the void, ii) is sufficiently great to permit passage therethrough of non-conglomerated blood cells, and iii) is sufficiently small to occlude passage therethrough of the thrombus. The height of the first passageway should be at least about about 12-16 micrometers, and is preferably at least about about 50 micrometers.

The width of the narrow passageway at the portion of the first step nearest the inlet region in the fluid path is greater than the height of the first passageway. Thus, when the fluid is passed from the inlet region to the outlet region of the device by way of the narrow passageway, the thrombus is sequestered on the inlet side of the first passageway. Preferably, the the width of the first passageway is at least 1,000 or 1,000,000 times the narrow dimension of the first passageway, to permit capture of multiple thrombi on the inlet side of the first passageway.

The separation element can have one or more subsequent steps on the outlet side of the first step. In such devices, the narrow passageway includes one or more corresponding subsequent sequential passageways fluidly connecting the first passageway and the outlet region. Each subsequent sequential passageway is bounded by the corresponding subsequent step and the surface of the void, and has a height defined by the distance between the corresponding subsequent step and the surface of the void. The height of each subsequent sequential passageway is preferably smaller than the height of the sequential passageway that precedes it. The width of the narrow passageway at the portion of each subsequent step nearest the inlet region in the fluid path is preferably greater (even more preferably 1,000 or 1,000,000 times greater) than the height of the subsequent sequential passageway. In such devices, thrombi of varying sizes can be captured at the upstream side of steps corresponding roughly in size to the sizes of the thrombi.

A second fluid can be passed from the inlet region into the outlet region by way of the narrow passageway. The second fluid can include an agent intended to aid in visualization or rupture of a thrombus sequestered within the device, such as a thrombolytic agent in an amount effective to degrade the thrombus.

The device and methods described herein can be used to remove thrombi from an animal's blood, such as that of a human. To achieve this, a blood sample (e.g., systemic blood) is taken from the animal, passed through the device, wherein fluid that reaches the outlet region has had any thrombi which occurred in the sample sequestered or broken up within the device. The resulting fluid can, for example, be administered to the same animal, be stored, or be used for other purposes. The void of the apparatus can also be examined to determine whether any thrombi present in the sample are sequestered therein, and this examination can reveal the presence of thrombi in the sample and facilitate further analysis or testing of the thrombi.

In addition to in vitro uses of the devices, some or all blood entering an upstream portion of a blood vessel of the animal can routed through the narrow passageway of the device (or through multiple devices connected in parallel) and fluid exiting the apparatus can be returned or administered to a downstream portion of the same blood vessel. In this mode of operation, the device can act essentially as a 'thrombus filter,' sequestering thrombi traveling through the blood vessel. The vessel can be one in which thrombi are known or believed to exert effects detrimental to health, such as one of a femoral vein, a carotid artery, a cerebral artery, a pulmonary artery, and a coronary artery.

### BRIEF DESCRIPTION OF THE SEVERAL VIEWS OF THE DRAWINGS

The foregoing summary, as well as the following detailed description of preferred embodiments of the invention, will be better understood when read in conjunction with the appended drawings. For the purpose of illustrating the invention, there is shown in the drawings embodiments which are presently preferred. However, that the invention is not limited to the precise arrangements and instrumentalities shown.

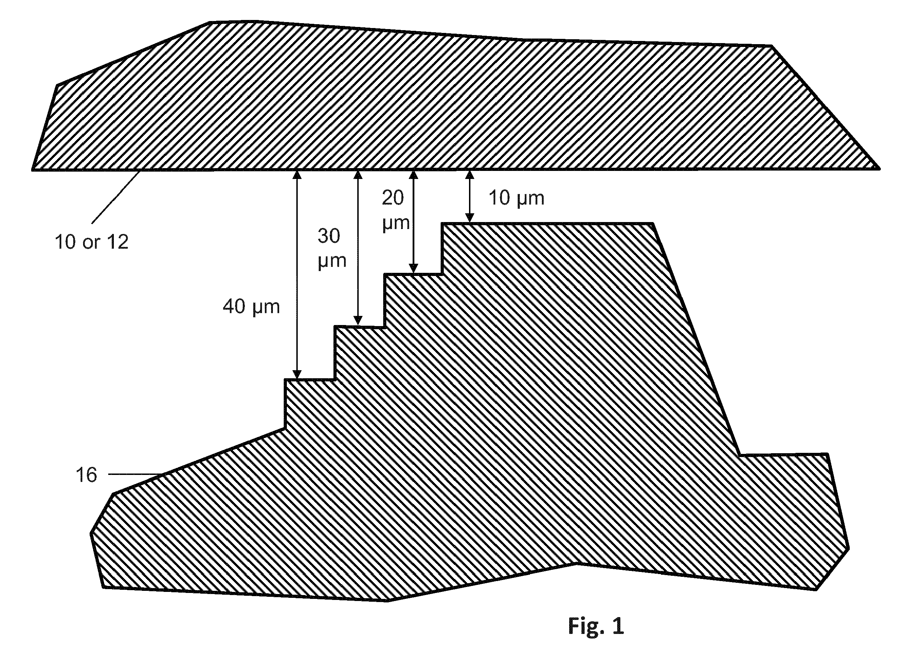

FIG. 1 is a cross section of a portion of the apparatus described herein, showing the stepped structure of the separation element. Numbers indicate relative distance between the separation element (16) and the cover (12) or body (10).

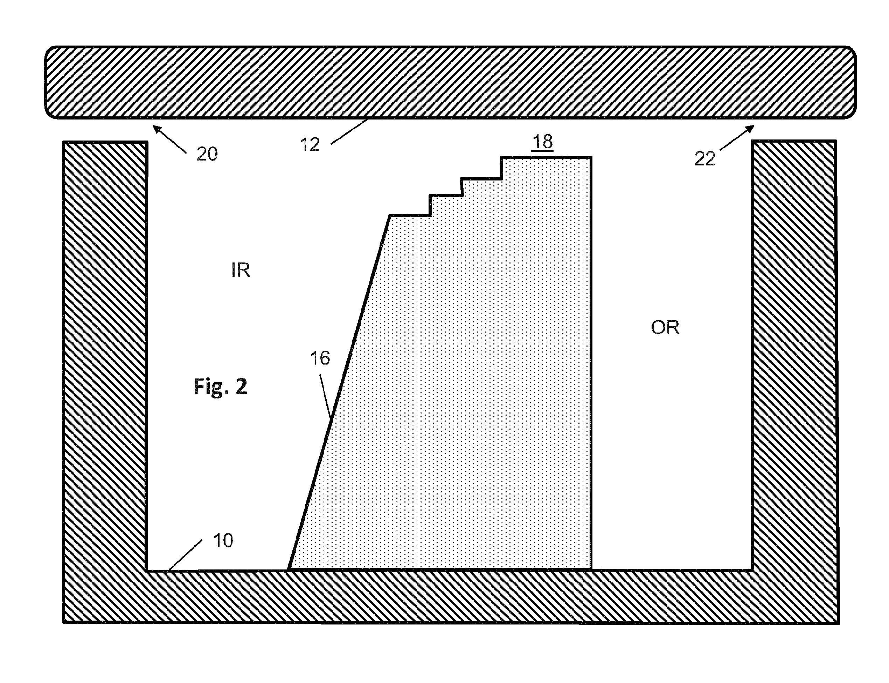

FIG. 2 is a cross section of an example of an assembled apparatus described herein, wherein the separation element (16) is opposed against the body (10), has steps of various heights on the face facing the inlet region (IR), opposite the outlet region (OR). A narrow passageway (18) exists between the highest step and the cover (12). An inlet port (20) and an outlet port (22) are shown.

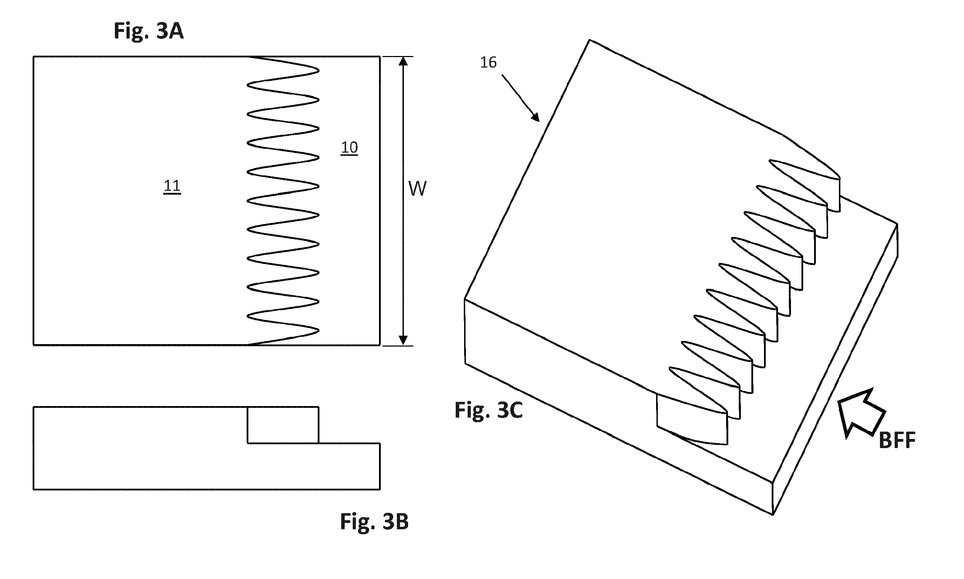

Figure 3 consists of Figures 3A, 3B, and 3C, which are, respectively, top, side and orthogonal views of a separation element (16) such as that illustrated in Figure 2, but consisting of only two steps, a first step (10) and a second step (11). The direction of bulk fluid flow (BFF, i.e., from the inlet region toward the separation element) is shown in Figure 3C. The upstream face of the first step is substantially planar in this embodiment, while the upstream face of the second step has an undulating configuration, with the result that the upstream face and leading edge of the second step each have a substantially greater length than the length of the upstream face of the first step, even though the first and second steps can be installed within a single passage of uniform width (W).

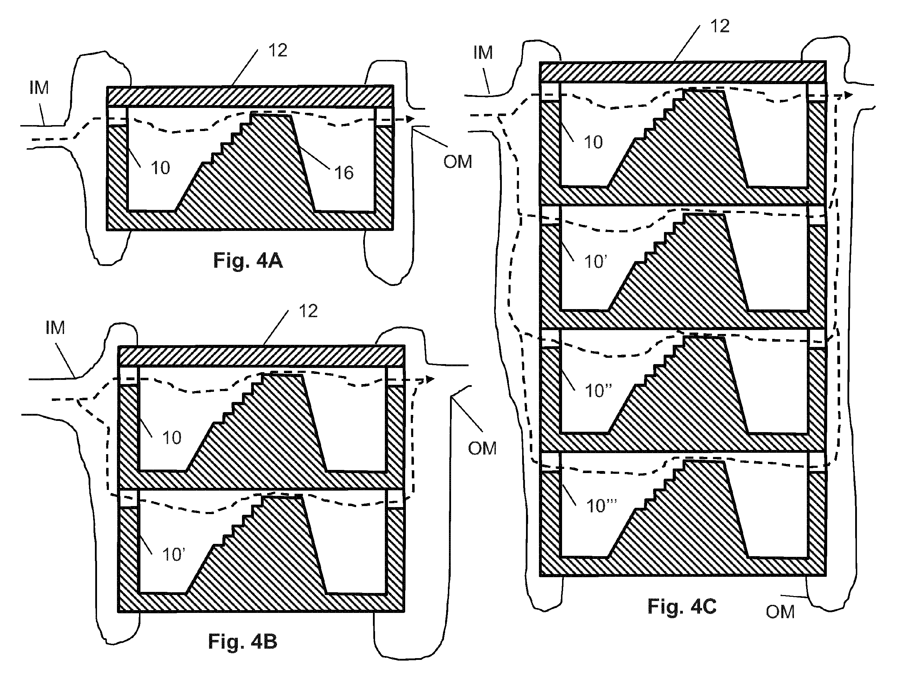

Figure 4 consists of Figures 4A, 4B, and 4C, each of which illustrates a thrombus capture device described herein fluidly linked with an inlet manifold (IM) and an outlet manifold (OM). The direction of bulk fluid flow is indicated with a dashed line(s) extending between IM and OM. The device illustrated in Figure 4A consists of a cover (12) fixed against a single body (10) having an integral separation element (16) interposed between IM and OM in the void formed between the body (10) and cover (12), forming a narrow passageway. The device illustrated in Figure 4B includes the components shown in Figure 4A and also includes a second body (10') fixed against a portion of body (10), thereby forming a second parallel flow path having a second narrow passageway. Fluid passing between IM and OM can flow through either narrow passageway. The device illustrated in Figure 4C includes the components shown in Figure 4B and also includes a third body (10") fixed against a portion of body (10') and a fourth body (10'") fixed against a portion of body (10"), thereby forming third and fourth parallel flow paths having a third and fourth narrow passageways. Fluid passing between IM and OM can flow through any of the four narrow passageways.

### DETAILED DESCRIPTION OF THE INVENTION

The invention relates to use of an apparatus for sequestering thrombi and emboli that are present in a blood sample. In particular, sequestration of thrombi and emboli formed of cross-linked blood platelets, extracellular humoral coagulation factors, other cells, and combinations of these can be achieved by passing a blood sample through a narrow passageway in the device in order to sequester thrombi and emboli on the 'upstream' (i.e., inlet) side of the narrow passageway, while permitting some or all of the other components of the blood to traverse the narrow passageway.

An embolus forms when all or a portion of a thrombus detaches from its point of attachment and floats freely in the circulation. Although thrombi tend to remain fixed at particular positions when they form in the circulatory system of an animal, thrombi can move within blood vessels by detaching, moving 'downstream' in the direction of blood flow, and re-attaching to the vessel wall. Rather than trying to distinguish between movement of emboli and thrombi, the term "thrombus" and its grammatical forms is used to encompass both thrombi and emboli for the remainder of this disclosure, except where context clearly requires otherwise.

The apparatus described herein can be used to capture thrombi present in a sample, including in blood withdrawn from a subject, such as human or other animal blood. The capture results from inability of thrombi to traverse the stepped separation element in the device when thrombus-containing blood passes through it. A thrombus is retained on the upstream side of the separation element until and unless the thrombus breaks up into pieces sufficiently small to traverse the narrowest portion of the flow passage. Multiple thrombi can be captured on the upstream side of the separation element, with the upper limit being determined by the width of the upstream edge (i.e., the leading edge) of the separation element. So long as the flow passage is not completely occluded by thrombi at the portion adjacent the leading edge of the separation element, fluid flow through the apparatus can continue and additional thrombi can be captured at the leading edge of the separation element in the non-occluded portion of the flow passage. Consequently, increasing the width (i.e., the dimension perpendicular to the height of the flow passage and perpendicular to the direction of bulk fluid flow) of the flow passage will tend to increase the capacity of the apparatus for capturing thrombi. Separation elements that have undulating or serpentine configurations, such as those described in copending U.S. application 14/077,811, can have a leading edge that is much greater in length than the width of the narrow passageway (e.g. 10² to 10⁶ times larger). Such separation elements exhibit a capacity to retain, without clogging, thrombi that are unable to traverse the narrow passageway than are separation elements having a less involuted leading edge. Similarly, configuring the separation element in a spiral conformation (optionally having an undulating or sinusoidal shape along the edge of the spiral) will tend to increase the capacity of the apparatus.

In the figures and several descriptions of the device presented in this disclosure and documents referred to herein, the device is described in terms of planar devices, such as a glass slide and a cover slip such as might be used with a microscope, with the slide having a void therein containing the separation element, the narrow passage being formed between the separation element and one or more of the slide and slip. Such devices can be readily fabricated using known techniques, and have the disadvantage of being relatively non-compact.

Compactness of the device can be improved by constructing the device using curved or rolled materials, while preserving the relevant dimensions and proportions described herein. By way of example, a relatively non-compact device can be made by laying out upon a 3-inch by 1-inch standard microscope slide a device in which a highly involuted separation element separates inlet and outlet regions of the slide. Even if the slide and a corresponding cover slip are very thin, the non-compact device still occupy a 3 x 1 inch rectangular area. However, if the slide and slip (with the separation element and fluid passages etched upon one of them) were made of a flexible material, the assembled device could be rolled up along its long (3 inch) axis to form a cylinder roughly 1 inch tall and having a diameter determined by the thickness of the slide and slip to yield a relatively compact device. The thickness of that compact device could be further reduced by omitting the slip and using the face of the slide opposite that upon which the separation element and fluid passages are etched in place of the cover slip to form a boundary of the narrow passageway (the separation element forming the opposite boundary). In such ways, devices having a rolled, folded, crumpled, or other nonplanar configuration can operate as described herein, so long as the thrombus-segregating capacity of the separation element and the flow capacity of the narrow passageway are preserved. Similarly, devices having a generally planar configuration can be stacked (i.e., the flat surface of one device acting as the cover for the separation-element and fluid-passageway-bearing surface of a second such device to yield a block of elements having a flow-through capacity equal to the sum of the capacities of the individual stacked devices, as shown in Figure 4, for example. Devices requiring folding or bending during assembly must be made using materials having the requisite flexibility, while rigid materials can be used to make device components not folded, flexed, or bent.

Break-up of thrombi can occur simply by passing blood through a separation device having thrombi captured therein. The mechanism by which thrombus break-up occurs is not fully understood, and likely corresponds to the mechanism by which thrombi naturally break up in the bloodstream. Thrombus decomposition can be accelerated by providing to the lumen of the separation device an anticoagulant (e.g., heparin or warfarin), a thrombolytic agent, or a fluid (e.g., blood or saline) which lacks activated clotting factors. Break-up can also be hastened by modulating fluid flow through the apparatus (e.g., by applying bulk fluid flow rate that vary in a pulsatile fashion). Thrombi which do not break up following capture by the separation device will tend to be retained at or near the upstream edge of the separation element. Even if a thrombus does not break up following its capture by the device, retention of thrombi within the device can beneficially protect tissues downstream of the device from ischemic conditions which the thrombus might otherwise have induced therein.

If the fluid throughput capacity of the separation apparatus is sufficiently great, then the device can be maintained in-line in the bloodstream. In such an embodiment, the thrombus-capture capacity of the apparatus should be selected so that accumulation of thrombi in the apparatus does not reduce throughput below the normal volumetric flow rate of blood at the site of insertion. All, or only a portion, of blood flowing through a vessel can be shunted into an in-line apparatus.

In-line insertion of a thrombus-capture apparatus in the bloodstream of a patient can be useful to protect thrombus-sensitive tissues in situations in which thrombus formation can be reasonably anticipated. It is known that certain patients are at greater risk than others of developing thrombi within their circulatory systems, and the apparatus described herein can be attached to or implanted within such patients before or after thrombus formation has been confirmed. Medical complications arising from vascular occlusion by thrombi can be alleviated or prevented by installing a thrombus-capture device in a blood vessel between a thrombus-generating site and a sensitive site. By way of examples:

Many strokes and TIAs are believed to be attributable to movement of thrombi (and especially shedding of emboli from pre-existing thrombi) within the vascular system to cerebral blood vessels having lumens too small to accommodate their passage. Upon occlusion of such a cerebral blood vessel, ischemic conditions can develop in cerebral tissue supplied by the vessel, leading to pathological neurologic symptoms which can be temporary (in the case of TIAs) or permanent (in the case of strokes). Capture of thrombi prior to occlusion of cerebral blood vessels could prevent these conditions. As captured thrombi decompose or break up, their component parts can pass through cerebral blood vessels if the height of the narrow passageway of the apparatus described herein is selected to correlate with the luminal diameter of those vessels (or if the height of the narrow passageway is selected to be smaller than that diameter). Because much blood supply to the brain passes through the carotid arteries, implantation of apparatus as described herein in-line or in parallel with one or more carotid arteries can capture thrombi which might otherwise cause strokes or TIAs. If one or more apparatus is installed in line with a carotid artery, the fluid throughput capacity of the apparatus should be selected to readily accommodate blood flow rates necessary for normal cerebral function, even when the apparatus has captured a foreseeable number of thrombi. Alternatively or in addition, an openable shunt can be installed which directs blood flow around the apparatus in the event it becomes clogged. As yet another alternative or addition, the separation device described herein can have multiple discrete narrow passages connected in parallel (or multiple of the devices may be connected in parallel), so that throughflow can continue even if a thrombus captured in one of the narrow passages promotes further thrombosis sufficient to occlude most or all of that narrow passage. This can be of particular significance in an indwelling device or in a device that is maintained in connection with blood for a period of many minutes, hours, or days.

Many PEs are believed to be attributable to movement of thrombi (and especially shedding of emboli from pre-existing thrombi) within the vascular system to pulmonary blood vessels (primarily pulmonary arteries and their branches and arterioles within the lung) having lumens too small to accommodate passage of the thrombi. Upon occlusion of such a pulmonary blood vessel, ordinary blood gas exchange is inhibited or prevented in lung tissue supplied by the vessel, leading to pathological neurologic symptoms which can be annoying or uncomfortable (e.g., shortness of breath) or serious (e.g., unconsciousness or sudden death). Although thrombi capable of occluding pulmonary blood vessels can arise in many body locations, their formation in and shedding from veins of the leg are known to be common sources of pulmonary emboli, especially in patients known to be afflicted with deep vein thrombosis. Implantation of apparatus as described herein in-line or in parallel with one or more veins of the legs and trunk (e.g., a femoral vein) can capture thrombi which might otherwise lead to PE. If one or more apparatus is installed in line with a femoral or other vein, the fluid throughput capacity of the apparatus should be selected to readily accommodate venous blood flow rates at ordinary venous blood pressure, even when the apparatus has captured a foreseeable number of thrombi. Alternatively or in addition, an openable shunt can be installed which directs blood flow around the apparatus in the event it becomes clogged with thrombi.

Many CEs are believed to be attributable to movement of thrombi (and especially shedding of emboli from pre-existing thrombi) within the vascular system to cardiac blood vessels (primarily coronary arteries) having lumens too small to accommodate passage of the thrombi. Upon occlusion of such a cardiac blood vessel, oxygen supply to cardiac muscle and nervous tissues is inhibited or prevented, leading to pathological symptoms which can be annoying or uncomfortable (e.g., chest pain, shortness of breath, or fatigue) or serious (e.g., unconsciousness, arrhythmia, or cardiac arrest). Although thrombi capable of occluding coronary arteries can arise in many body locations, their formation in and shedding from thrombotic plaques in the left cardiac ventricle are known to be common sources of cardiac emboli, especially in patients known to be afflicted with heart pathologies such as atrial fibrillation. Implantation of apparatus as described herein in-line or in parallel with one or more coronary arteries can capture thrombi which might otherwise lead to CE. If one or more apparatus is installed in line with a coronary artery, the fluid throughput capacity of the apparatus must be selected to readily accommodate cardiac blood flow rates at foreseeable elevated cardiac rates, even when the apparatus has captured a foreseeable number of thrombi. Alternatively or in addition, an openable shunt can be installed which directs blood flow around the apparatus in the event it becomes clogged with thrombi.

### The Apparatus

The terms "device" and "apparatus" are used synonymously in this disclosure.

The apparatus comprises a body having a void therein. The void has an inlet region and an outlet region and can be filled with fluid. A separation element separates the inlet and outlet regions of the void and has at least one step with a characteristic height. Inclusion of multiple steps of different heights (i.e., corresponding to decreasing narrow passageway height along the path of fluid flow) can increase the thrombus-capture capacity of the device, presumably by sequestering thrombi of different sizes at different portions of the device. A cover is disposed across the void, and covers at least the portion of the void wherein the highest portion of the separation element occurs. The cover can be permanently or non-permanently opposed against matching portions of the body (i.e., adhered to or fused with it or removably urged against it). Fluid flow through the device passes from the inlet region, across and over the separation element, and into the outlet region. Particles, such as thrombi, which have a size larger than the dimensions of the narrow passageway defined by the steps of the separation element and the opposed portion of the body or cover are unable to pass through the narrow passageway and are sequestered or captured within the lumen of the device, at least until and unless the break up into smaller pieces able to pass through the narrow passageway.

Developments in methods of manufacturing very small devices, such as microelectronic devices, have made it possible to precisely and reproducibly make devices having features with nanometer-scale dimensions. Devices having structural elements with minimal dimensions ranging from micrometers to tens or hundreds of micrometers (i.e., a range of sizes which span the sizes of individual human cells and conglomerations of cells) have been described, such as in U.S. Patent 7,993,908, U.S. patent publication number 2011/0065181, and U.S. patent application 14/077,811. Those or other methods can be used to make the apparatus described herein.

In one embodiment, the cover is disposed across substantially the entire area of the void, yielding a closed fluid system. The cover, the body, or both, can have inlet and an outlet ports. The ports can be simple holes which extend through the cover or body, or they can have fixtures (burrs, rings, hubs, or other fittings) associated with them for facilitating connection of a fluid handling device with the port. These ports facilitate addition and withdrawal of fluid and facilitate passage of blood into the apparatus or passage of thrombus-depleted blood therefrom.

The shape, material, and construction of the body are not critical, except that the void in the body should be formed or machined in such a way that a cover can be applied across the void in order to form a fluid-tight seal with the body around the edges of the void. Preferably, the void is formed in a flat portion of the body, and a flat cover is used that has a size and shape sufficient to completely cover the void.

Similarly, the shape, material, and construction of the cover are not critical, except that the cover should form a fluid-tight seal with the body at the edges of the void and should extend from the body at one edge of the void, along the highest portion of the separation element across the void, to the body at another edge of the void, thereby defining (with the separation element) a narrow passageway through which liquid must flow in order to pass from the inlet region to the outlet region of the void.

The separation element has a 'stepped' structure with at least two steps. One of the steps is the highest. The separation element can be attached to (or integral with) either the body or the cover, or it can be a separate piece of material sandwiched between the body and the cover. When the device is assembled, the steps of the separation element define selected distances between the separation element and either the body (i.e., the surface of the void in the body) or the cover. Assessed in the direction from the inlet region of the void toward the outlet region of the void, the distance between the steps and the body or cover decreases. Thus, movement of particles such as thrombi suspended in a fluid (e.g., blood) flowing through the assembled apparatus can be inhibited or halted at a step that is characteristic of a dimension of the thrombus. The distance between the step and the cover or body defines the height of the narrow passageway of the apparatus at that position. The face of a step can be substantially parallel to the face of the body or cover, so that the height of the narrow passageway is substantially constant across the width of the step. Alternatively, the face of a step can be non-parallel to the body/cover, so that the height of the narrow passageway varies across the width of the step. Such a step will permit passage of different-sized particles across its width, and may be less prone to clogging by thrombi captured at the step.

Substantially any particles that have characteristic dimensions on the same order as the distance between the steps and the cover or body can be captured using the apparatus. For thrombus capture applications, it is desirable that the apparatus have a narrow passageway that permits passage of substantially all non-coagulated components of blood (i.e., individual blood cells of all types) but has a height sufficiently small to capture multi-cell particles such as thrombi. The precise height selected can depend on the luminal diameter of blood vessels situated downstream from the apparatus (because it is occlusion of those vessels that is desired to be avoided). Generally speaking, the height should be sufficiently small to capture thrombi having a size sufficient to occlude blood vessels or to otherwise induce ischemic damage at tissues supplied by such vessels. By way of example, an apparatus having a narrow passageway height in the range 10-20 micrometers is believed to be effective to permit passage of normal blood cells, but to occlude passage through the apparatus of thrombi. A narrow passageway height of 16-20 micrometers is also appropriate. An apparatus in which the narrow passageway height is greater than about 50 or 100 micrometers throughout the flow path may be undesirable, in that it may permit passage of thrombi likely to induce ischemic damage attributable to their likelihood of occluding capillary beds. Generally speaking, even in devices with multiple steps, the height of the narrowest narrow passageway should be sufficient to permit passage of normal components of blood, and steps should be included to capture thrombi of known or anticipated size within the device. Devices with multiple steps defining narrow passageways of various heights, for example, can be used to capture thrombi of varying sizes at different areas within the device (e.g., capturing thrombi having dimensions on the order of millimeters at an upstream area while capturing thrombi having dimensions on the order of tens of micrometers at a downstream area).

The steps of the separation element can be discrete steps. Alternatively, one or more of the steps can be sloped, the steps being separated from one another by a flat portion that extends in the direction from the inlet region toward the outlet region. The length (in the direction of fluid flow) of the flat portion is not critical. The flat portions of different steps can have the same length, or they can have different lengths.

The body, the cover, or both, can have an optical, electrical, or optico-electrical device constructed therein or thereon (e.g., by etching, film deposition, or other known techniques) at a position that corresponds to a selected step or region of a step. Such devices can be used to detect thrombi (e.g., using a detector to detect a decrease in light or other radiation transmitted across the fluid between the surface of the step and the cover or body) or to manipulate thrombi captured on the step.

Provision of a fluid to a selected step (or a plurality of selected steps) can be performed by adding fluid at that step by way of a fluid channel formed in the apparatus. In such a way, a fluid containing an antithrombotic, an anti-coagulant, a dye, or another agent capable of interacting with a thrombus can be brought into contact with a thrombus captured by the apparatus.

When the apparatus is filled with fluid, the fluid fills the void, including the inlet and outlet regions, and completely covers the separation element. If desired, the apparatus can be lightly manipulated (e.g., by tilting or inverting), or the fluid can be applied under pressure, in order to ensure that all portions of the void are filled with the fluid. If necessary, any air bubbles that may be present can generally be removed by applying pressurized fluid to the inlet, the outlet, or alternately to the inlet and outlet to dislodge them, pressurized fluid optionally being applied in a pulsatile fashion. Such bubbles can also be removed by permitting the gas to dissolve into a fluid (e.g., a de-aerated fluid) provided to the void or by passing a fluid having a high solubility limit for the gas(es) (e.g., an ethanol-water mixture for atmospheric air bubbles) through the void. Especially if the apparatus is to be implanted into an animal, air bubbles should be removed therefrom prior to implantation.

The body, cover, and separation element can be constructed from substantially any material that will hold its shape during operation of the apparatus as described herein. However, rigid materials are preferred, at least for devices that are assembled by stacking or opposing components against one another. Examples of suitable materials include various glasses, solid polymers, and crystalline minerals. Silicon is a preferred substrate material because of the well-developed technology permitting its precise and efficient fabrication, but other materials can be used, including various glasses and cast, molded, or machined polymers including polytetrafluoroethylenes. The inlet and outlet ports, the separation element, and the surfaces defining the void in the body can be fabricated inexpensively in large quantities from a silicon substrate by any of a variety of micromachining methods known to those skilled in the art. The micromachining methods available include film deposition processes such as spin coating and chemical vapor deposition, laser fabrication or photolithographic techniques such as UV or X-ray processes, precision machining methods, or etching methods which may be performed by either wet chemical processes or plasma processes. (See, e.g., Manz et al., 1991, Trends in Analytical Chemistry, 10:144-149). Surfaces of the device can be treated with an anticoagulant (e.g., heparin) in order to inhibit or prevent thrombosis which might otherwise be induced upon contact of blood with the surface.

Steps of varying widths and heights can be fabricated with microscale dimensions for separating cells in a sample. A silicon substrate containing a fabricated steps can be covered and sealed (e.g., anodically bonded) with a thin glass or plastic cover. Other clear or opaque cover materials may be used. Alternatively, two silicon substrates can be sandwiched, or a silicon substrate can be sandwiched between two glass covers. Preferably, at least one of the body and the cover is transparent. Use of a transparent material facilitates dynamic viewing of the contents of the device, and allows probing of fluid flow in the apparatus, either visually or by machine. Other fabrication approaches can be used.

The surfaces of the apparatus can be chemically treated or coated with any of a variety of known materials which reduce or enhance agglutination of cells with the material selected for the cover, body, or obstacles. By way of example, an antibody which binds specifically with a cell-surface antigen can be attached to a surface of the void using any of a variety of protein anchoring chemistries, a surface of a step, or a surface of the cover, in order to inhibit passage of cells which exhibit the antigen past the surface (e.g., in order to differentiate cells of similar size but different type). The surfaces of the apparatus can also be treated with any of a variety of known reagents (e.g., oxygen plasma) in order to increase the hydrophilicity of the surfaces. This treatment can improve the rate and completeness of filling of the apparatus with a fluid medium introduced into the apparatus and can decrease the likelihood of individual blood cells adhering to the apparatus or coagulating thereon.

One advantage of the apparatus described herein is that they can be manufactured in a wide variety of sizes and geometrical arrangements, depending on the intended use of the apparatus. In addition, multiple apparatus can be manufactured on or in a single piece of material, such as a unitary silicon or plastic block or a microscope slide. Furthermore, the multiple apparatus on a slide can be connected in series, in parallel, or both. By way of example, several apparatus having a narrow passageway of relatively small height (e.g., 2 micrometers) can be constructed on a single block of material, and a sample (e.g., blood) can be fed to the inlet region of each of those apparatus. By feeding fluid through the apparatus, blood cells can pass through the device, while thrombi and other large particles can be removed from the sample.

After thrombi have been removed, the flow in the apparatus can be reversed, and the retained thrombi can be recovered, if desired.

The cover, body, and separation element (if not already connected to one of the cover and body) can be provided in the form of a kit to be assembled by the user (e.g., after adding a fluid medium to the void in the body). The kit can also include instructions for using the apparatus or reagents to be used therewith. The apparatus can be supplied pre-filled with fluid.

The apparatus can have indicia associated in a fixed position with respect to the separation element. The indicia can be used to assess whether thrombi having a selected characteristic (e.g., size or ability to bind with an antibody fixed to a surface of the apparatus) are being retained in the apparatus. The indicia can be printed, painted, or stamped on, or engraved or etched in the body or the cover, preferably on a surface of a component that is transparent, so that the indicia and the thrombi in the apparatus can be simultaneously observed by a user. The indicia preferably do not alter the shape, diameter, or smoothness of the fluid path with which they are associated. For example, the indicia can be on or in the opposite face of a transparent material in which the fluid path exists. Alternatively, the indicia can be on or in one face of a transparent material that has a different face opposed against the fluid path (e.g., the exterior face of the cover).

The apparatus is used to remove thrombi from a blood sample in the following way. A blood sample is provided to the inlet region of the apparatus. Fluid flow through the device is thereafter initiated or continued. The height of the narrow passageway at the separation element is sufficiently great to permit passage therethrough of non-conglomerated blood cells and sufficiently small to occlude passage therethrough of conglomerated or agglutinated blood cells, such as a thrombus. Because conglomerated blood cells are unable to pass through the narrow passageway (at least if the size of the conglomerate exceeds the height of the narrow passageway), they are sequestered at the separation element or on the inlet side of it. Blood lacking the occluded conglomerate(s) reaches the outlet region of the apparatus and can flow therefrom, be withdrawn therefrom, or be forced therefrom by fluid flow behind it.

The width of the narrow passageway at the portion of the separation element nearest the inlet region in the fluid path should be greater than the height of the narrow passageway, so that capture of a single thrombus does not occlude fluid flow therethrough. However, if many narrow passageways are arranged in parallel in the apparatus, the width of each narrow passageway is less critical, since occlusion of one or some of them does not preclude continued use of the other narrow passageways. Preferably, the width of the narrow passageway is at least 1,000 times, 10,000 times, 100,000 times, or 1,000,000 times the height (i.e., the narrow dimension) of the first passageway.

The apparatus described herein may be used to remove thrombi from blood taken from an animal (e.g., a human blood sample) for a number of purposes. Thrombi can be removed prior to returning the blood to the same animal or to a different animal (preferably of the same species, such as in stored human blood units commonly used in medical practice). Alternatively, the apparatus may be installed in line in a blood vessel of an animal, such that all blood flow through the vessel passes through the apparatus, for the purpose of removing substantially all thrombi from the passing blood while permitting fragments of captured thrombi to return to the blood as a captured thrombus breaks up. In yet another alternative, the apparatus may be installed in parallel to a blood vessel of the animal, such that only a portion of the blood passing through the vessel is passed through the device (to remove thrombi therefrom) prior to returning the portion to the vessel.

The apparatus can also be used for diagnostic purposes. That is, the apparatus can be used to separate thrombi from a blood sample for the purpose of identifying the presence of the thrombi, without regard to whether the thrombus-depleted blood is returned to the animal from which it was obtained. Such diagnostic methods are performed substantially the same way as described herein. A blood sample is passed through the apparatus, causing thrombi to be sequestered within it if they are unable to traverse the narrow passageway of the apparatus. The apparatus can thereafter be examined for the presence of captured thrombi. Such examination can be effected by disassembling the apparatus, by looking at a transparent portion of it, by operating a detector associated with it (as described herein), or in any other fashion in which the presence of a captured thrombus can be detected.

It will be appreciated by those skilled in the art that changes could be made to the embodiments described above without departing from the broad inventive concept thereof. It is understood, therefore, that this invention is not limited to the particular embodiments disclosed, but covers modifications within the spirit and scope of the present invention as defined by the appended claims.

While the subject matter has been disclosed herein with reference to specific embodiments, it is apparent that other embodiments and variations of this subject matter can be devised by others skilled in the art without departing from the true spirit and scope of the subject matter. The appended claims include all such embodiments and equivalent variations.

The features disclosed in the foregoing description, in the following claims and/or in the accompanying figures may, both separately and in any combination thereof, be material for realising the invention in diverse forms thereof.

### CLAUSES

The present invention will now be described with reference to the following clauses:
1. A method of sequestering a thrombus in a blood sample, the method comprising
   providing the blood sample to the inlet region of an apparatus, the apparatus comprising a body, a cover, and a separation element,
   the body and cover defining a void having an inlet region, an outlet region, and a surface,
   the separation element i) being disposed in the void, ii) having at least one step including a first step, and iii) defining a narrow passageway that fluidly connects the inlet and outlet regions in a fluid path,
   the narrow passageway including at least a first passageway,
   the first passageway fluidly being bounded by the first step and the surface of the void, and having a height defined by the distance between the first step and the surface of the void,
   the height of the first passageway being sufficiently great to permit passage therethrough of non-conglomerated blood cells and sufficiently small to occlude passage therethrough of the thrombus, and
   the width of the narrow passageway at the portion of the first step nearest the inlet region in the fluid path being greater than the height of the first passageway, and
   passing a first fluid from the inlet region into the outlet region by way of the narrow passageway, whereby the thrombus is sequestered on the inlet side of the first passageway.
2. The method of clause 1, wherein the width of the first passageway is at least 1,000 times the narrow dimension of the first passageway.
3. The method of clause 1, wherein the width of the first passageway is at least 1,000,000 times the narrow dimension of the first passageway.
4. The method of clause 1, wherein the separation element has at least one subsequent step on the outlet side of the first step,
   whereby the narrow passageway includes at least one subsequent sequential passageway fluidly connecting the first passageway and the outlet region, each subsequent sequential passageway being bounded by the corresponding subsequent step and the surface of the void, and having a height defined by the distance between the corresponding subsequent step and the surface of the void,
   the height of each subsequent sequential passageway being smaller than the height of the sequential passageway that precedes it.
   the width of the narrow passageway at the portion of each subsequent step nearest the inlet region in the fluid path being greater than the height of the subsequent sequential passageway,
5. The method of clause 1, wherein at least one of a surface of the void and a surface of the separation element has an anticoagulant associated therewith.
6. The method of clause 1, wherein the body and the cover are unitary.
7. The method of clause 6, wherein the body is wrapped about itself in a spiral configuration and wherein the void is defined between opposite faces of the body.
8. The method of clause 1, further comprising passing a second fluid from the inlet region into the outlet region by way of the narrow passageway, the second fluid comprising a thrombolytic agent in an amount effective to degrade the thrombus.
9. The method of clause 1, wherein the first fluid and the blood sample are the same fluid.
10. The method of clause 1, wherein the first fluid is substantially free of cells prior to its passage into the inlet region.
11. The method of clause 1, wherein the height of the first passageway is at least about about 12-16 micrometers
12. The method of clause 1, wherein the height of the first passageway is at least about about 50 micrometers.
13. The method of clause 1, wherein the blood sample is systemic blood taken from an animal and wherein fluid that reaches the outlet region is administered to the same animal.
14. The method of clause 13, wherein the animal is a human.
15. The method of clause 1, wherein all blood entering an upstream portion of a blood vessel of the animal is routed through the narrow passageway of at least one of the apparatus and wherein all fluid exiting the apparatus is administered to a downstream portion of the blood vessel.
16. The method of clause 15, wherein the blood vessel is selected from the group consisting of a femoral vein, a carotid artery, a cerebral artery, a pulmonary artery, and a coronary artery.
17. The method of clause 15, wherein all blood entering an upstream portion of a blood vessel of the animal is routed through a unit comprising a plurality of the apparatus connected in parallel.
18. The method of clause 17, wherein individual units within the apparatus are separately replaceable.
19. A method of assessing the presence of a thrombus in a blood sample, the method comprising
   sequestering the thrombus according to the method of clause 1 and thereafter observing the portion of the void upstream from the first step to detect the thrombus therein.
20. The method of clause 19, wherein the portion of the void is observed microscopically.
21. The method of clause 19, wherein the presence of the thrombus in the portion of the void is observed by measuring transmittance of light through at least one portion of the void.
22. In a method of treating a patient at risk for a thrombotic disorder, the improvement comprising assessing the presence of a thrombus in the patient's blood using the method of clause 19 and administering a thrombolytic agent to the patient if a thrombus is detected therein.
23. A method of reducing the likelihood that a patient will experience a thrombotic disorder, the method comprising
   installing into the circulation of the patient an apparatus comprising a body, a cover, and a separation element,
   the body and cover defining a void having an inlet region, an outlet region, and a surface,
   the separation element i) being disposed in the void, ii) having at least one step including a first step, and iii) defining a narrow passageway that fluidly connects the inlet and outlet regions in a fluid path,
   the narrow passageway including at least a first passageway,
   the first passageway fluidly being bounded by the first step and the surface of the void, and having a height defined by the distance between the first step and the surface of the void,
   the height of the first passageway being sufficiently great to permit passage therethrough of non-conglomerated blood cells and sufficiently small to occlude passage therethrough of the thrombus, and
   the width of the narrow passageway at the portion of the first step nearest the inlet region in the fluid path being greater than the height of the first passageway,
   blood from the patient being provided to the inlet region of the apparatus and fluid present at the outlet region of the apparatus being administered into the bloodstream of the patient.

## Claims

1. A method of sequestering a thrombus in a blood sample, the method comprising providing the blood sample to the inlet region of an apparatus, the apparatus comprising a body, a cover, and a separation element,
the body and cover defining a void having an inlet region, an outlet region, and a surface,
the separation element i) being disposed in the void, ii) having at least one step including a first step, and iii) defining a narrow passageway that fluidly connects the inlet and outlet regions in a fluid path,
the narrow passageway including at least a first passageway,
the first passageway fluidly being bounded by the first step and the surface of the void, and having a height defined by the distance between the first step and the surface of the void,
the height of the first passageway being sufficiently great to permit passage therethrough of non-conglomerated blood cells and sufficiently small to occlude passage therethrough of the thrombus, and
the width of the narrow passageway at the portion of the first step nearest the inlet region in the fluid path being greater than the height of the first passageway, and
passing a first fluid from the inlet region into the outlet region by way of the narrow passageway, whereby the thrombus is sequestered on the inlet side of the first passageway.

2. The method of claim 1, wherein the width of the first passageway is: at least 1,000 times the narrow dimension of the first passageway; OPTIONALLY,
at least 1,000,000 times the narrow dimension of the first passageway.

3. The method of claim 1 or claim 2, wherein the separation element has at least one subsequent step on the outlet side of the first step,
whereby the narrow passageway includes at least one subsequent sequential passageway fluidly connecting the first passageway and the outlet region, each subsequent sequential passageway being bounded by the corresponding subsequent step and the surface of the void, and having a height defined by the distance between the corresponding subsequent step and the surface of the void,
the height of each subsequent sequential passageway being smaller than the height of the sequential passageway that precedes it,
the width of the narrow passageway at the portion of each subsequent step nearest the inlet region in the fluid path being greater than the height of the subsequent sequential passageway.

4. The method of any one of claims 1-3, wherein at least one of a surface of the void and a surface of the separation element has an anticoagulant associated therewith.

5. The method of any one of claims 1-4, wherein the body and the cover are unitary; AND/OR, wherein the body is wrapped about itself in a spiral configuration and wherein the void is defined between opposite faces of the body.

6. The method of any one of claims 1-5, further comprising passing a second fluid from the inlet region into the outlet region by way of the narrow passageway, the second fluid comprising a thrombolytic agent in an amount effective to degrade the thrombus.

7. The method of any one of claims 1-6, wherein the first fluid and the blood sample are the same fluid; AND/OR,
wherein the first fluid is substantially free of cells prior to its passage into the inlet region.

8. The method of any one of claims 1-7, wherein the height of the first passageway is: at least about about 12-16 micrometers; OPTIONALLY,
at least about about 50 micrometers.

9. The method of any one of claims 1-8, wherein the blood sample is systemic blood taken from an animal and wherein fluid that reaches the outlet region is administered to the same animal; OPTIONALLY, wherein the animal is a human.

10. The method of any one of claims 1-9, wherein all blood entering an upstream portion of a blood vessel of the animal is routed through the narrow passageway of at least one of the apparatus and wherein all fluid exiting the apparatus is administered to a downstream portion of the blood vessel; OPTIONALLY,
wherein the blood vessel is selected from the group consisting of a femoral vein, a carotid artery, a cerebral artery, a pulmonary artery, and a coronary artery; AND/OR,
wherein all blood entering an upstream portion of a blood vessel of the animal is routed through a unit comprising a plurality of the apparatus connected in parallel; OPTIONALLY,
wherein individual units within the apparatus are separately replaceable.

11. A method of assessing the presence of a thrombus in a blood sample, the method comprising
sequestering the thrombus according to the method of any one of claims 1-10 and thereafter
observing the portion of the void upstream from the first step to detect the thrombus therein.

12. The method of claim 11, wherein: the portion of the void is observed microscopically; AND/OR, the presence of the thrombus in the portion of the void is observed by measuring transmittance of light through at least one portion of the void.

13. A thrombolytic agent for use in treating a patient if a thrombus is present in the patient's blood, the use comprising assessing the presence of a thrombus in the patient's blood using the method of any one of claims 11 or 12.

14. Treated blood obtained by:
providing blood from a patient to an apparatus comprising a body, a cover, and a separation element,
the body and cover defining a void having an inlet region, an outlet region, and a surface,
the separation element i) being disposed in the void, ii) having at least one step including a first step, and iii) defining a narrow passageway that fluidly connects the inlet and outlet regions in a fluid path,
the narrow passageway including at least a first passageway,
the first passageway fluidly being bounded by the first step and the surface of the void, and having a height defined by the distance between the first step and the surface of the void,
the height of the first passageway being sufficiently great to permit passage therethrough of non-conglomerated blood cells and sufficiently small to occlude passage therethrough of the thrombus, and
the width of the narrow passageway at the portion of the first step nearest the inlet region in the fluid path being greater than the height of the first passageway, and,
passing the blood from the inlet region from the inlet region into the outlet region by way of the narrow passageway.

15. The treated blood of claim 14 for use in treating, or reducing the likelihood the patient will experience, a thrombotic disorder.
